(19) Europäisches Patentamt
European Patent Office
Office européen des brevets

(11) **EP 4 233 732 A1**

(12) **EUROPEAN PATENT APPLICATION**

(43) Date of publication:
**30.08.2023   Bulletin 2023/35**

(21) Application number: **22165641.6**

(22) Date of filing: **30.03.2022**

(51) International Patent Classification (IPC):
**A61B 10/00** (2006.01)      **A61F 13/38** (2006.01)
**C12M 1/30** (2006.01)       **G01N 1/02** (2006.01)
**B01L 3/00** (2006.01)        A61B 10/02 (2006.01)

(52) Cooperative Patent Classification (CPC):
**A61B 10/0045; A61B 10/02; B01L 3/5029;**
**C12M 33/02;** A61B 2010/0216; A61F 13/38;
B01L 2200/12; G01N 2001/028

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **28.02.2022   KR 20220026080**

(71) Applicant: **PMW Corp.**
**Busan 48059 (KR)**

(72) Inventors:
• **Park, Jeong Hyeop**
  **47532 Yeonje-gu, Busan (KR)**
• **Yun, Jung Yong**
  **47563 Yeonje-gu, Busan (KR)**

(74) Representative: **Gulde & Partner**
**Patent- und Rechtsanwaltskanzlei mbB**
**Wallstraße 58/59**
**10179 Berlin (DE)**

(54) **SWAB FOR COLLECTING SAMPLE AND METHOD FOR PREPARING THEREOF**

(57)   Disclosed herein are a swab for collecting a sample, which can provide a remarkably improved sample extraction efficiency by planting a swab pile of 0.5 to 2.5 deniers into a swab support, and can be mass-produced efficiently and sanitarily without agglomeration of cilia, and a manufacturing method thereof.

**FIG.1**

EP 4 233 732 A1

Description

## BACKGROUND OF THE INVENTION

### Field of the Invention

The present invention relates to a swab for collecting a sample and a manufacturing method thereof, and more particularly, to a swab for collecting a sample, which can provide a remarkably improved sample extraction efficiency by planting a swab pile of 0.5 to 2.5 deniers into a swab support, and can be mass-produced efficiently and sanitarily without agglomeration of cilia, and a manufacturing method thereof.

### Background Art

In general, a swab is used to collect mucus or tissues from a part of the nose, mouth, ear, etc. of a person, and is used for sample collection to diagnose infection of pathogens or viruses in a hospital.

Recently, a corona virus (COVID-19) having a fast transfer rate by aerial infection and a very high mortality rate is going around the world. Also, in Korea, a great deal of infected persons occur, and a number of the death by COVID-19 is being reported. So, tests for confirming infection of the corona virus are carried out in hospitals or health care centers of the country.

In order to test infection of the corona virus, first, a sample must be collected from a testee. A swab for collecting a sample is inserted into the testee's nasal cavity or oral cavity where infection occurs actually in order to collect rheum from a mucous membrane of the inner wall of the nasal cavity or the oral cavity.

In fact, in order to collect a sample from the nasal cavity, a tester inserts the swab for collecting a sample into the nasal cavity to get in contact with the inner wall of the nasal cavity from which the most rheum is obtained, and collects a sample of rheum enough to see with eyes while rotating the swab for collecting a sample.

Moreover, in order to collect a sample from the oral cavity, in a state in which a testee opens the mouth wide, a tester inserts the swab for collecting a sample into the oral cavity to get in contact with the tonsil surface of the inner wall of the nasal cavity or with the inside of the pharyngolarynx from which the most rheum is obtained while pressing the testee's tongue with a tongue depressor, and collects a sample of rheum enough to see with eyes while rotating the swab for collecting a sample.

However, a conventional swab has a disadvantage in that it is difficult to collect a large amount of sample since cilia for collecting a sample are wound like a cotton swab that we usually use. Moreover, the conventional swab has another disadvantage in that a tester has to collect a sample several times due to the length of the short swab since the swab must be deeply inserted into the oral cavity or the nasal cavity if a sample is not collected.

Furthermore, since the conventional swab has the cilia formed such that cotton or fabric is covered at an end of a connection rod manually, the conventional swab is difficult to automate and depends on manpower, and thus, productivity is reduced, a failure rate is high since the standard of the cilia is not accurate, and a production cost is high.

Therefore, in order to supplement the above-mentioned disadvantages, the present inventors have completed the present invention since recognizing that it is urgent to prevent a tangle of the cilia and by preventing the entanglement of cilia and to develop a method of manufacturing an efficient swab.

## PATENT LITERATURE

### Patent Documents

Patent Document 1: Korean Patent Publication No. 10-2015-0087656
Patent Document 1: Korean Patent No. 10-2283281

## SUMMARY OF THE INVENTION

Accordingly, the present invention has been made in view of the above-mentioned problems occurring in the prior art, and it is an object of the present invention to provide a swab for collecting a sample, which has a swab pile of 0.5 to 2.5 deniers planted into a swab support and provides a remarkably improved sample extraction efficiency without agglomeration of cilia.

It is another object of the present invention to provide a manufacturing method of a swab for collecting a sample, which can be mass-produced efficiently and sanitarily without agglomeration of cilia since the swab pile of 0.5 to 2.5 deniers is charged with electricity and the cilia are flocked to a swab support coated with an adhesive.

The technical problem to be solved by the present invention is not limited to the technical problem as mentioned above, and another technical problem, which is not mentioned, could be clearly understood by those having ordinary skill in the art to which the present invention pertains based on the description below.

To accomplish the above object, according to the present invention, there are provided a swab for collecting a sample and a method for manufacturing the same.

Hereinafter, the present invention will be described in detail.

In an aspect of the present invention, the present invention provides a swab for collecting a sample including: a swab support; and a swab pile formed at one side of the swab support.

In an embodiment of the present invention, the swab pile has a thickness of 0.5 to 2.5 deniers and a length of 0.8 to 1.0 mm.

In another aspect of the present invention, the present invention provides a manufacturing method of a swab for collecting a sample, including the steps of:

(S1) manufacturing a flocking jig by coating an adhesive on the swab support;
(S2) performing electric charge to the swab pile; and
(S3) planting a swab pile into one side of the swab support.

In an embodiment of the present invention, the step (S1) includes the steps of:

(S1A) coating an adhesive on one side of the swab support; and
(S1B) fixing the swab support, on which the adhesive is coated, to the flocking jig.

In an embodiment of the present invention, the step (S3) includes the steps of:

(S3A) positioning the flocking jig and the swab pile between a first high-pressure plate and a second high-pressure plate;
(S3B) applying voltage of 15 to 45 kV and current of 1 to 5 mA to the first high-pressure plate, and applying voltage of 15 to 45 kV to the second high-pressure plate; and
(S3C) planting the swab pile into one side of the swab support using a potential difference between the first high-pressure plate and the second high-pressure plate.

In an embodiment of the present invention, the manufacturing method of a swab for collecting a sample further includes the step of (S4) drying the swab support in which the swab pile is planted.

In an embodiment of the present invention, the step (S4) is a step of drying the swab support, in which the swab pile is planted, at temperature of 55 to 65°C and in a humidity condition of 20 to 80%.

All matters described in the swab for collecting a sample and the manufacturing method thereof are applied in the same way if they are not contradictory.

The swab for collecting a sample according to an embodiment of the present invention has a swab pile of 0.5 to 2.5 deniers planted into a swab support, and provides a remarkably improved sample extraction efficiency without agglomeration of cilia.

Additionally, the manufacturing method of a swab for collecting a sample is capable of mass-producing the swabs efficiently and sanitarily without agglomeration of cilia since the swab pile of 0.5 to 2.5 deniers is charged with electricity and the cilia are flocked to a swab support coated with an adhesive.

The effects of the present invention are not limited to the above-mentioned effects and further effects not described above will be clearly understood by those skilled in the art.

## BRIEF DESCRIPTION OF THE DRAWINGS

The above and other objects, features and advantages of the present invention will be apparent from the following detailed description of the preferred embodiments of the invention in conjunction with the accompanying drawings, in which:

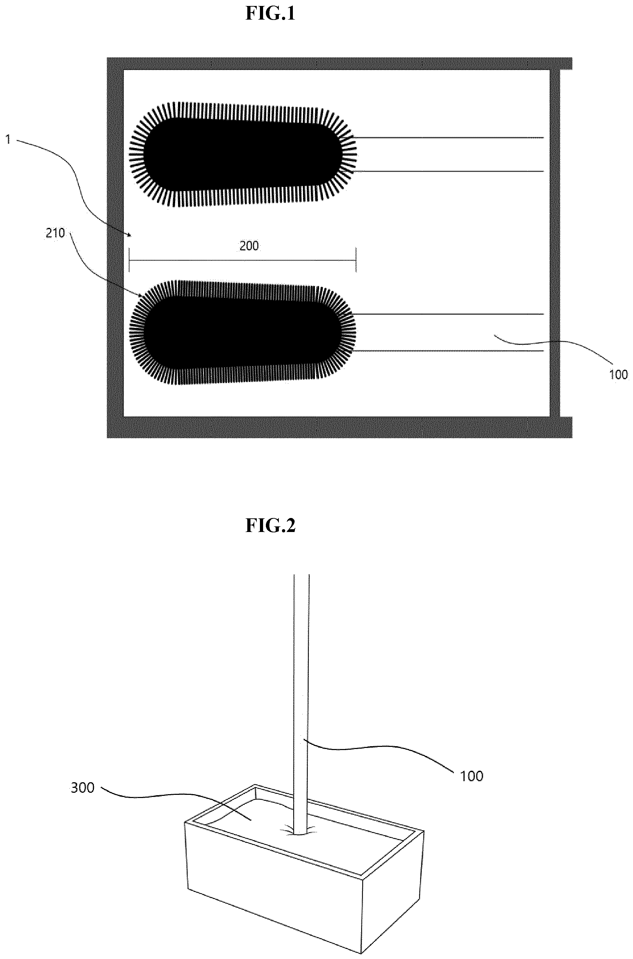

FIG. 1 is a schematic front view illustrating a swab for collecting a sample according to the present invention;
FIG. 2 is a schematic view illustrating a step of coating an adhesive on one side of a swab support in a manufacturing method of a swab for collecting a sample according to the present invention;

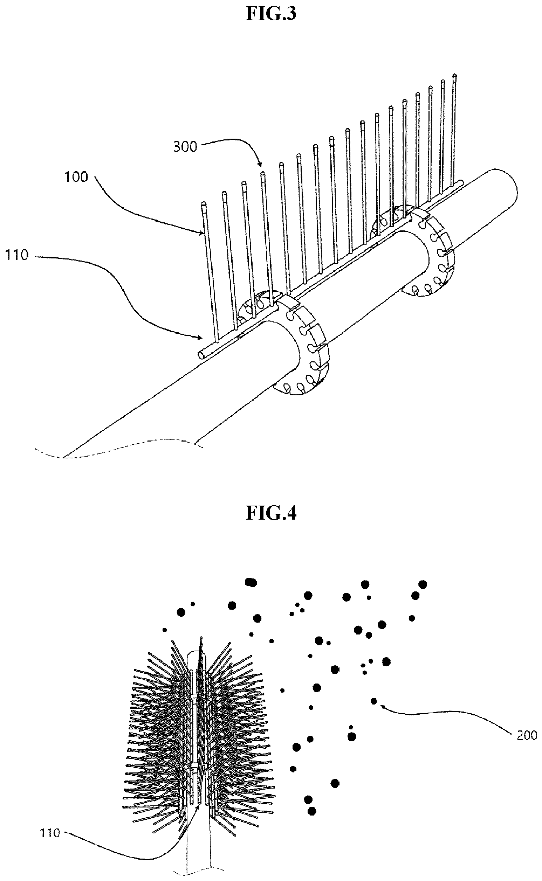

FIG. 3 is a schematic view illustrating a step of fixing the swab support coated with the adhesive to a flocking jig in the manufacturing method of a swab for collecting a sample according to the present invention;

FIG. 4 is a schematic view illustrating a step of planting a swab pile into one side of the swab support through high pressure treatment in the manufacturing method of a swab for collecting a sample according to the present invention;
FIG. 5 illustrates measurement of the swab pile of the swab for collecting a sample through a scanning electron microscope (SEM); and
FIG. 6 illustrates a swab (embodiment 1) for collecting a sample, which is manufactured according to the present invention, and a comparative swab (comparative example 2) for collecting a sample.

## DETAILED DESCRIPTION OF THE PREFERRED EMBODIMENT

Terms including descriptive or technical terms which are used herein should be construed as having meanings that are obvious to one of ordinary skill in the art. However, the terms may have different meanings according to an intention of one of ordinary skill in the art, precedent cases, or the appearance of new technologies. Also, some terms may be arbitrarily selected by the applicant, and in this case, the meaning of the selected terms will be described in detail in the detailed description of the invention. Thus, the terms used herein have to be defined based on the meaning of the terms together with the description throughout the specification.

Unless otherwise defined, all terms, including technical and scientific terms, used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and/or the present application, and will not be interpreted in an idealized or overly formal sense unless expressly so defined herein.

The numerical range includes the numerical values defined in the range. All maximum numerical limits given throughout this specification include all lower numerical limits as if the lower numerical limits were clearly written. All minimum numerical limits given throughout this specification include all higher numerical limitations as if the higher numerical limit were clearly written. All numerical limits given throughout this specification will include all better numerical ranges within the broader numerical ranges, as if narrower numerical limits are clearly written.

Hereinafter, embodiments of the present invention will be described in detail, but the protection scope of the present invention is not limited to the following embodiments.

### Swab for collecting sample

The present invention provides a swab 1 for collecting a sample, comprising: a swab support 100; and a swab pile part 200 formed on one side of the swab support 100.

The swab support 100 is made of a resin material, and is at least one selected from the group consisting of polyoxymethylene (POM), polystyrene (PS), polypropylene (PP), polyethylene (PE), polyurethane (PU), polyamide (PA), polycarbonate (PC), high density polyethylene (HDPE), low density polyethylene (LDPE), polymethyl methacrylate (PMMA), polyvinyl chloride (PVC), and acrylonitrile butadiene styrene copolymer resin (ABS resin).

The swab pile part 200 may be one or more swab piles 210 selected from the group consisting of nylon, rayon, polypropylene, polyester, polyamide, and acrylic.

The swab pile part 200 can be formed on one side of the swab support 100, and is preferably located on the upper portion of the swab support 100.

The swab pile part 200 can have a thickness of 0.5 to 2.5 deniers. The swab pile part 200 of the swab according to the present invention shows higher sample extraction efficiency when collecting a sample since having the thickness of 0.5 to 2.5 deniers.

The term "denier" used in the present invention is a measure of a thickness of a yarn, and the denier applied to the present invention means a thickness of the swab pile of the present invention.

The swab pile part 200 can have a length of 0.8 to 1.0 mm. If the length of the swab pile part 200 is less than 0.8 mm, the swab pile part 200 holds together on the swab support 100 due to a warp phenomenon when the swab 1 for collecting a sample is manufactured. If the length of the swab pile part 200 exceeds 1.0 mm, lumpishness may occur during drying when the swab 1 for collecting a sample is manufactured. Therefore, it is preferable that the swab pile part 200 be 0.8 to 1.0 mm.

In the swap 1 for collecting a sample, the swab support 100 and the swab pile part 200 can be formed at a length ratio of 3:1 to 12:1 (swab support to swab pile part), and the swab support 100 and the swab pile part 200 are formed at a thickness ratio of 0.1:1 to 0.6:1 (swab support to swab pile part), thereby improving structural stability.

In addition, the swab 1 for collecting a sample according to the present invention can be a swab for the nasal cavity or the oral cavity.

The swap 1 for collecting a sample according to the present invention has the swab pile of 0.5 to 2.5 deniers planted into a swab support, thereby providing a remarkably improved sample extraction efficiency without agglomeration of cilia.

**Manufacturing method of swab for collecting sample**

The present invention provides a manufacturing method of a swap 1 for collecting a sample, comprising the following steps:

(S1) manufacturing a flocking jig 110 by coating an adhesive 300 on the swab support 100;
(S2) performing electric charge to the swab pile 210; and
(S3) planting a swab pile 210 into one side of the swab support 100.

The swap 1 for collecting a sample is as described above.
The step (S1) is a step of coating an adhesive 300 on the swab support 100 to manufacture a flocking jig 110, and includes the steps:

(S1A) coating an adhesive 300 on one side of the swab support 100; and
(S1B) fixing the swab support 100, on which the adhesive 300 is coated, to the flocking jig 110.

In more detail, the step (S1A) is a step of coating an adhesive 300 on one side of the swab support 100. The swab support 100 can be coated with the adhesive 300 when being dipped in a mold containing the adhesive 300 therein.
The adhesive 300 is a mixed adhesive of an acrylic monomer and urethane, and the acrylic monomer may be methyl methacrylate and methacrylic acid.
Furthermore, the adhesive 300 may have a viscosity of 18,000 to 25,000 cps at 25°C, and preferably, has a viscosity of 18,000 to 22,000 cps at 25°C.
In addition, the adhesive 300 may be a neutral range of 7 pH to 8.5 pH or may be weakly basic.
The adhesive 300 may be coated to have a thickness ranging from 0.20 mm to 1.00 mm from the surface of the swab support 100. More specifically, if the adhesive 300 is coated on the surface of the swab support 100 to be less than 0.20 mm, the swab pile 210 may not be fixed. If the adhesive 300 is coated on the surface of the swab support 100 to exceed 1.00 mm, the swab pile 210 is injected into the adhesive 300 so deeply that the swab pile 210 gets too shorter in the swab 1 for collecting a sample according to the present invention. Therefore, it is most preferred that the adhesive 300 be coated to have a thickness of 0.20 to 1.00 mm from the surface of the swab support 100.
The step (S2) is a step of performing electric charge to the swab pile 210. Silica, a metallic material, or tannin is coated on the surface of the swab pile 210. In more detail, since silica, a metallic material, or tannin is coated on the surface of the swab pile 210, the swab pile 210 can be fixed to the swab support by being charged during high-pressure treatment, which will be described later, it can prevent the swab pile 210 from yellowing through the charging process.
In the step (S3), a swab pile 210 is planted into one side of the swab support 100 to manufacture a swap 1 for collecting a sample, and includes the following steps of:

(S3A) positioning the flocking jig 110 and the swab pile 210 between a first high-pressure plate and a second high-pressure plate;
(S3B) applying voltage of 15 to 45 kV and current of 1 to 5 mA to the first high-pressure plate, and applying voltage of 15 to 45 kV to the second high-pressure plate; and
(S3C) planting the swab pile 210 into one side of the swab support 100 using a potential difference between the first high-pressure plate and the second high-pressure plate.

In the step (S3), high pressure is applied to the first high-pressure plate and the second high-pressure plate, and the swab pile 210 can be more strongly planted into one side of the swab support 100 by the potential difference generated at the same time.
The step (S3) may be performed at temperature of 15 to 40°C and in a humidity condition of 20 to 80%.
Moreover, the manufacturing method of the swap 1 for collecting a sample may further include a step of: (S4) drying the swab support 100 in which the swab pile 210 is planted to form a swab pile part 200.
In more detail, the step (S4) may be the step of: (S4) drying the swab support 100, in which the swab pile 210 is planted to form the swab pile part 200, at temperature of 55 to 65°C and in a humidity condition of 20 to 80%.
The manufacturing method of the swap 1 for collecting a sample according to the present invention is capable of mass-producing the swabs efficiently and sanitarily without agglomeration of cilia since the swab pile of 0.5 to 2.5 deniers is charged with electricity and the cilia are flocked to the swab support 100 coated with the adhesive 300.
Advantages and features of the present disclosure and methods accomplishing the advantages and features will become apparent from the following detailed description of exemplary embodiments with reference to the accompanying drawings. However, the present invention is not limited to exemplary embodiment disclosed herein but will be implemented in various forms. The exemplary embodiments are provided so that the present invention is completely disclosed, and a person of ordinary skilled in the art can fully understand the scope of the present invention. Therefore, the present invention will be defined only by the scope of the appended claims.

### Embodiment 1. Manufacturing of swab for collecting sample

A swab support made of polyoxymethylene (POM) was dipped in a mixed adhesive of acrylic monomer (methylmethacrylate and methacrylic acid) and urethane (21,000 cps, 7 pH, and 25°C) to be coated by a thickness of 0.57 mm, and a swab support was fixed to the flocking jig. Next, a swab pile which was made of a nylon material and had a thickness of 1.1 denier and a length of 0.9 mm was coated with tannin to be charged with electricity. In addition, the swab support fixed to the flocking jig and the swab pile were located between a first high-pressure plate, in which voltage of 20 kV and current of 2 mA flows, and a second high-pressure plate, in which voltage of 20 kV flows, and the swab pile was planted into one side of the swab support by a potential difference between the first high-pressure plate and the second high-pressure plate. Through high-pressure treatment, the swab support in which the swab pile was planted was dried at temperature of 60°C and under humidity condition of 45%, thereby manufacturing a swab for collecting a sample according to the present invention.

### Comparative example 1. Manufacturing of comparative swab 1 for collecting sample

All conditions were the same as in the Embodiment 1, except that a comparative swab 1 for collecting a sample was manufactured such that a swap pile of the comparative swab had a thickness of 3.0 deniers.

### Comparative example 2. Manufacturing of comparative swab 2 for collecting sample

All conditions were the same as in the Embodiment 1, except that a comparative swab 2 for collecting a sample was manufactured such that an adhesive coated on a swap support had a thickness of 1.5 mm.

### Comparative example 3. Manufacturing of comparative swab 3 for collecting sample

All conditions were the same as in the Embodiment 1, except that a comparative swab 3 for collecting a sample was manufactured such that a swap pile was planted in a state where only a first high-pressure plate existed without a second high-pressure plate.

### Experimental example 1

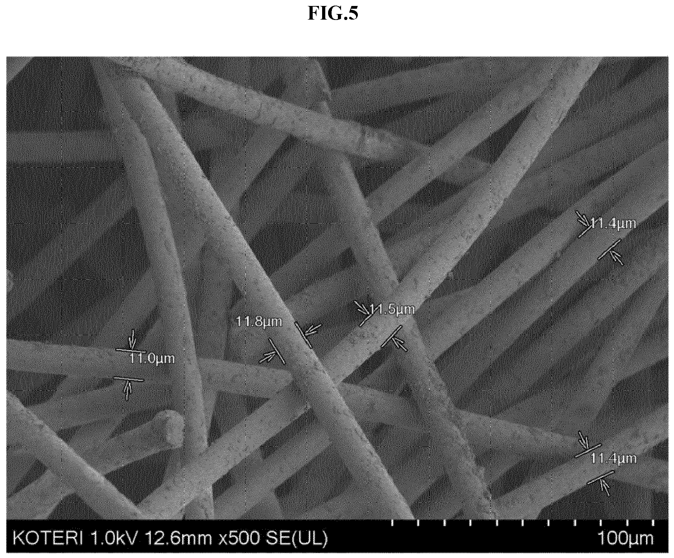

In order to confirm manufacturing of the swab for collecting a sample with 1.1 deniers, the swab manufactured in the Embodiment 1 was measured with a scanning electron microscope (SEM), and the measured result is illustrated in FIG. 5.
Referring to FIG. 5, it is confirmed that the swab for collecting a sample with 1.1 deniers was manufactured.

### Experimental example 2

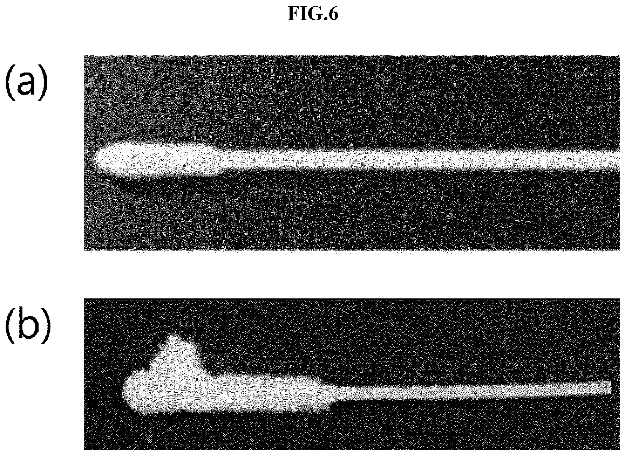

In the swab with 1.1 deniers, in order to confirm a swap manufacturing result according to the thickness of the coated adhesive, the swabs manufactured by the Embodiment 1 and the Comparative example 2 were checked, and the check results are illustrated in FIG. 6.
Referring to FIG. 6, FIG. 6(A) shows that the swab with 1.1 deniers according to the present invention was manufactured in the form that the swab pile was dispersed evenly, but FIG. 6(B) shows that the comparative swab 2 having the adhesive coated thick caused lumpishness of the swab pile.
Through the results, it was confirmed that the thickness of the adhesive coated on one side of the swab support according to the present invention was optimized in planting the swab pile evenly.

### Experimental example 3. Extraction efficiency

In order to confirm sample extraction efficiency of the swab for collecting a sample according to the present invention, as shown in the following [Table 1], a sample solution and an extracting solution were prepared. The swab pile of the swab manufactured in the Embodiment 1 was soaked in the sample solution for ten seconds, and then, the swab (Embodiment 1) absorbing the sample solution was soaked in the extracting solution. Then, the sample solution absorbed into the swab was dissociated in the extracting solution, and light with the wavelength of 595 nm was irradiated to the dissociated extracting solution three times in order to investigate absorbance. After that, sample extraction efficiency of the swab (Embodiment 1) according to the present invention was confirmed through the following [Mathematical Equation 1]. In order to compare the sample extraction efficiency, the same test was performed using the existing swab (Comparative example 1) having the swab pile of 3.0 deniers, and the test result is shown in the following [Table 2].

※ Positive control: Extracting solution

※ Negative control: Buffer solution

[Table 1]

| Solution | Composition |
|---|---|
| Sample solution | Mixture of artificial saliva (A.S.) and trypan blue of 0.4% (dye solution) (which were mixed at a volume ratio of 3:1) |
| Extracting solution | Mixture of sample solution and buffer solution (Tris buffer of 10 mM (8.0 pH) (which were mixed at a volume ratio of 1:5) |

[Mathematical Equation 1]

$$\frac{\text{Absorbance of dissociated extracting solution} - \text{Absorbance of negative control}}{\text{Absorbance of positive control} - \text{Absorbance of negative control}} \times 100 = \text{Extraction efficiency of swab}$$

[Table 2]

| No. | Embodiment 1 (Swab of 1.1 deniers) | Comparative example 1 (Swab of 3.0 deniers) |
|---|---|---|
| 1 | 97.5 | 85.9 |
| 2 | 96.4 | 85.7 |
| 3 | 96.3 | 85.1 |
| Average | 96.73 | 85.57 |

As confirmed in [Table 2], it is confirmed that the swab for collecting a sample according to the present invention has high sample extraction efficiency of 96.73%. Compared with the sample extraction efficiency of the existing swab having the swab pile of 3.0 deniers, the sample extraction efficiency of the swab according to the present invention is improved by 13% or more.

Through the above results, it shows that the swab for collecting a sample according to the present invention can provide a superior test result through high sample extraction efficiency when collecting bacteria, viruses, etc.

Those skilled in the art will understand that the present invention can be implemented as other concrete forms without changing the inventive concept or essential features. Therefore, it should be understood that the embodiments as described above are only proposed for illustrative purposes and do not limit the present invention.

**Claims**

1. A swab for collecting a sample comprising:

   a swab support; and
   a swab pile formed at one side of the swab support.

2. The swab according to claim 1, wherein the swab pile has a thickness of 0.5 to 2.5 deniers and a length of 0.8 to 1.0 mm.

3. A manufacturing method of a swab for collecting a sample comprising the steps of:

(S1) manufacturing a flocking jig by coating an adhesive on the swab support;
(S2) performing electric charge to the swab pile; and
(S3) planting a swab pile into one side of the swab support.

4. The manufacturing method according to claim 3, wherein the step (S1) comprises the steps of:

(S1A) coating an adhesive on one side of the swab support; and
(S1B) fixing the swab support, on which the adhesive is coated, to the flocking jig.

5. The manufacturing method according to claim 3, wherein the step (S3) comprises the steps of:

(S3A) positioning the flocking jig and the swab pile between a first high-pressure plate and a second high-pressure plate;
(S3B) applying voltage of 15 to 45 kV and current of 1 to 5 mA to the first high-pressure plate, and applying voltage of 15 to 45 kV to the second high-pressure plate; and
(S3C) planting the swab pile into one side of the swab support using a potential difference between the first high-pressure plate and the second high-pressure plate.

6. The manufacturing method according to claim 3, further comprising the step of:
(S4) drying the swab support in which the swab pile is planted.

7. The manufacturing method according to claim 6, wherein the step (S4) is a step of drying the swab support, in which the swab pile is planted, at temperature of 55 to 65°C and in a humidity condition of 20 to 80%.

## FIG.1

## FIG.2

EP 4 233 732 A1

## FIG.3

## FIG.4

10

FIG.5

KOTERI 1.0kV 12.6mm x500 SE(UL)                    100μm

**FIG.6**

(a)

(b)

Europäisches
Patentamt

European
Patent Office

Office européen
des brevets

# EUROPEAN SEARCH REPORT

**Application Number**

EP 22 16 5641

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 102 228 452 B1 (CHOI MYUNG GYU [KR]; SG MEDICAL INC [KR]) 16 March 2021 (2021-03-16) * paragraphs [0028] – [0081]; figures 1-6 * | 1-7 | INV. A61B10/00 A61F13/38 C12M1/30 G01N1/02 B01L3/00 |
| X | KR 102 247 929 B1 (KIM JONG WON [KR]) 4 May 2021 (2021-05-04) * paragraphs [0008] – [0059]; figures 1-5 * | 1-7 | ADD. A61B10/02 |
| X | US 2011/282243 A1 (NAKATANI HIROSHI [JP]) 17 November 2011 (2011-11-17) * paragraphs [0021] – [0047]; figures 1-3 * | 1-7 | |
| X | WO 2021/217025 A1 (UNIV CARNEGIE MELLON [US]) 28 October 2021 (2021-10-28) * paragraphs [0046] – [0068]; figures 1-15 * | 1-7 | |
| X | US 8 114 027 B2 (TRIVA DANIELE [IT]; COPAN INNOVATION LTD [IE]) 14 February 2012 (2012-02-14) * column 2, line 60 – column 4, line 61; figures 1,2 * | 1-7 | TECHNICAL FIELDS SEARCHED (IPC) A61B A61F C12M G01N B01L |
| X | EP 0 418 266 A1 (STORMBY NILS [SE]) 27 March 1991 (1991-03-27) * column 2, line 45 – column 5, line 50; figures 1-15 * | 1-7 | |

The present search report has been drawn up for all claims

| Place of search | Date of completion of the search | Examiner |
|---|---|---|
| Munich | 16 August 2022 | Hochrein, Marion |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EP 4 233 732 A1

### ANNEX TO THE EUROPEAN SEARCH REPORT
### ON EUROPEAN PATENT APPLICATION NO.

EP 22 16 5641

This annex lists the patent family members relating to the patent documents cited in the above-mentioned European search report.
The members are as contained in the European Patent Office EDP file on
The European Patent Office is in no way liable for these particulars which are merely given for the purpose of information.

16-08-2022

| Patent document cited in search report | | | Publication date | Patent family member(s) | | | Publication date |
|---|---|---|---|---|---|---|---|
| KR 102228452 | | B1 | 16-03-2021 | CN | 114441228 | A | 06-05-2022 |
| | | | | EP | 3995209 | A1 | 11-05-2022 |
| | | | | JP | 2022075481 | A | 18-05-2022 |
| | | | | KR | 102228452 | B1 | 16-03-2021 |
| | | | | US | 2022142620 | A1 | 12-05-2022 |
| KR 102247929 | | B1 | 04-05-2021 | NONE | | | |
| US 2011282243 | | A1 | 17-11-2011 | CN | 202173415 | U | 28-03-2012 |
| | | | | JP | 2011234666 | A | 24-11-2011 |
| | | | | US | 2011282243 | A1 | 17-11-2011 |
| WO 2021217025 | | A1 | 28-10-2021 | NONE | | | |
| US 8114027 | | B2 | 14-02-2012 | AU | 2004226798 | A1 | 14-10-2004 |
| | | | | CA | 2515205 | A1 | 14-10-2004 |
| | | | | DE | 202004021787 | U1 | 20-01-2011 |
| | | | | DE | 202004021907 | U1 | 18-06-2012 |
| | | | | DE | 202004021908 | U1 | 18-06-2012 |
| | | | | DE | 202004021930 | U1 | 12-11-2012 |
| | | | | DE | 202004021932 | U1 | 12-11-2012 |
| | | | | DE | 602004010240 | T2 | 25-09-2008 |
| | | | | DK | 1608268 | T3 | 25-03-2008 |
| | | | | EP | 1608268 | A1 | 28-12-2005 |
| | | | | ES | 2297406 | T3 | 01-05-2008 |
| | | | | JP | 4579902 | B2 | 10-11-2010 |
| | | | | JP | 2007523663 | A | 23-08-2007 |
| | | | | NZ | 541560 | A | 25-09-2009 |
| | | | | US | 2006142668 | A1 | 29-06-2006 |
| | | | | US | 2011028861 | A1 | 03-02-2011 |
| | | | | US | 2012150088 | A1 | 14-06-2012 |
| | | | | US | 2012271196 | A1 | 25-10-2012 |
| | | | | US | 2013072817 | A1 | 21-03-2013 |
| | | | | US | 2013338535 | A1 | 19-12-2013 |
| | | | | US | 2016045188 | A1 | 18-02-2016 |
| | | | | US | 2019307433 | A1 | 10-10-2019 |
| | | | | WO | 2004086979 | A1 | 14-10-2004 |
| EP 0418266 | | A1 | 27-03-1991 | AU | 616973 | B2 | 14-11-1991 |
| | | | | EP | 0418266 | A1 | 27-03-1991 |
| | | | | JP | H067830 | B2 | 02-02-1994 |
| | | | | JP | H04500320 | A | 23-01-1992 |
| | | | | KR | 900701223 | A | 01-12-1990 |
| | | | | SE | 463188 | B | 22-10-1990 |
| | | | | WO | 8910724 | A1 | 16-11-1989 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020150087656

- KR 102283281